# EUROPEAN PATENT APPLICATION

(11) **EP 3 061 577 A1**
(43) Date of publication of application: **31.08.2016**
(21) Application number: 14855848.9
(22) Date of filing: 17.10.2014
(51) Int. Cl.: B25J 13/02, A61B 1/00

(54) **MANIPULATOR SYSTEM CONTROL METHOD AND MANIPULATOR SYSTEM**

(30) Priority: 22.10.2013 US 201361894096 P; 23.01.2014 JP 2014010578
(71) Applicant: Olympus Corporation, Hachioji-shi Tokyo 192-8507 (JP)
(72) Inventor: OGAWA, Ryohei, Tokyo 192-8507 (JP); KISHI, Kosuke, Tokyo 192-8507 (JP)
(74) Representative: Gunzelmann, Rainer
(86) International application number: PCT/JP2014/077644
(87) International publication number: WO 2015/060207

(57) **Abstract**

Provided is a method of controlling a manipulator system that includes a manipulator having an end effector and a joint portion and a manipulation input device with which a movement direction of the end effector can be input via a manipulation member, wherein the joint portion has a first joint, which is pivotable about a first axis parallel to a direction in which the end effector and the joint portion are arranged, and a second joint, which is positioned further on the distal-end side than the first joint is and which is pivotable about a second axis perpendicular to the first axis, the manipulator-system controlling method including a receiving step (S2) of receiving the movement direction, a rotating step (S3) of actuating the first joint based on the movement direction, a flexing step (S6) of actuating the second joint based on the movement direction, and a shifting step (S5) of shifting from the rotating step (S3) to the flexing step (S6) in accordance with changes in the magnitude of a physical amount of manipulation applied to the manipulation member.

## Description

### {Technical Field}

The present invention relates to a manipulator-system controlling method and a manipulator system.

### {Background Art}

In the related art, there are known medical manipulator systems provided with a treatment tool that has an end effector and a joint portion and a manipulation input device that is manually manipulated by a surgeon (for example, see Patent Literatures 1 to 3). The surgeon can remotely manipulate the end effector inside a body by manipulating the joint portion by using the manipulation input device at hand. The manipulation input device of Patent Literature 1 is provided with a master arm having a joint structure analogous to the joint structure of the joint portion. Manipulation input devices of Patent Literatures 2 and 3 are provided with a joystick-style or a key-style manipulation member.

### {Citation List}

### {Patent Literature}

{PTL 1} Publication of Japanese Patent No. 5011060
{PTL 2} Japanese Unexamined Patent Application, Publication No. 2009-101077
{PTL 3} Japanese Unexamined Patent Application, Publication No. Hei 10-262900

### {Summary of Invention}

### {Technical Problem}

However, with a treatment tool that has, sequentially from the base side thereof, a rotary joint, which can be rotated about a longitudinal axis of the treatment tool, and a flexural joint which can be flexed in a direction that intersects the longitudinal axis, in some cases, depending on the attitude of the rotary joint, it is not possible to directly move the end effector in a desired direction just by actuating the flexural joint.

In such a case, with the manipulation input device of Patent Literature 1, it is necessary to actuate both the rotary joint and the flexural joint in combination, and the surgeon must calculate the actuation directions and the actuation amounts of these joints. There is a problem in that such work is complicated and requires well-developed skills. In addition, there is a problem in that, when a plurality of joints are simultaneously actuated by using a manipulation input device having an structure analogous to that of the treatment tool, the amount by which the master arm is operated is increased, thus making it difficult to reduce the size of the manipulation input device.

On the other hand, the manipulation input devices of Patent Literatures 2 and 3 are suitable for size-reduction thereof. However, in association with rotation of the rotary joint, the correspondence relationship between the flexing direction of the flexural joint of the treatment tool displayed on a display and the direction input via the manipulation member at hand changes. As a result, consistency related to directions of input and output is lost, and there is a problem in that, because the operator cannot obtain feedback for the manipulation direction of the manipulation member, it is not possible to intuitively manipulate the end effector.

The present invention has been conceived in light of the above-described circumstances, and an object thereof is to provide a manipulator-system controlling method and a manipulator system with which, in a manipulator system provided with a manipulator having a rotary joint and a flexural joint in this order from the base side, it is possible to intuitively manipulate an end effector in any direction just by a simple manipulation and it is also possible to reduce the size of a manipulation input device. {Solution to Problem}

In order to achieve the above-described object, the present invention provides the following solutions.

A first aspect of the present invention is a method of controlling a manipulator system that is provided with a manipulator that has an end effector and a joint portion, in this order from a distal-end side, and a manipulation input device with which a movement direction of the end effector can be input, wherein the joint portion has a first joint, which is pivotable about a first axis parallel to a direction in which the end effector and the joint portion are arranged, and a second joint, which is positioned further on the distal-end side than the first joint is and which is pivotable about a second axis perpendicular to the first axis, and wherein the manipulation input device has a manipulation member that can be manipulated by an operator, and the movement direction of the end effector is input by manipulating the manipulation member, the method of controlling the manipulator system including a receiving step of receiving the movement direction from the manipulation input device; a rotating step of actuating the first joint based on the movement direction received in the receiving step; a flexing step of, following the rotating step, actuating the second joint based on the movement direction received in the receiving step; and a shifting step of shifting from the rotating step to the flexing step in accordance with changes in a magnitude of a physical amount of a manipulation applied to the manipulation member.

With the first aspect of the present invention, when the operator manipulates the manipulation member, the movement direction corresponding to that manipulation is received in the receiving step, the pivoting direction of the second joint is aligned with the movement direction in the rotating step via actuation of the first joint, and, subsequently, the end effector is moved in the movement direction in the flexing step via actuation of the second joint. By doing so, regardless of the direction of the input movement direction, the end effector can be moved in the movement direction.

In this case, in the shifting step, the joint to be actuated is switched from the first joint to the second joint in accordance with the magnitude of the physical amount of the manipulation applied to the manipulation member. Specifically, the operator applies a manipulation corresponding to the movement direction of the end effector to the manipulation member, and, subsequently, he/she can intuitively move the end effector in a desired direction by a simple manipulation of merely changing the physical amount (for example, time, distance, pressure) of that manipulation. In addition, because it suffices that the manipulation input device allow at least the movement direction of the end effector to be input, the configuration thereof can be simplified, and the size thereof can be reduced.

In the above-described first aspect, in the receiving step, the movement direction corresponding to a two-dimensional position at which the manipulation is applied to the manipulation member or a direction in which the manipulation is applied to the manipulation member may be received, and, in the shifting step, the shift to the flexing step from the rotating step may be made accordance with changes in the physical amount of the manipulation applied at the same position or in the same direction.

By doing so, the first joint and the second joint are sequentially actuated by the operator merely by manipulating the same position of the manipulation member or by manipulating the manipulation member in the same direction. Specifically, the surgeon can move the end effector by hardly moving the hand placed on the manipulation member.

In the above-described first aspect, the physical amount may be an amount of time during which the manipulation member is being manipulated or an amount of movement of the manipulation member.

By doing so, the first joint and the second joint are sequentially actuated by the operator by continuing a manipulation that has once been applied to the manipulation member or by changing the amount of movement of the manipulation member. By doing so, it is possible to further reduce the movement of the hand of the surgeon required to move the end effector.

The above-described first aspect may include a flexing-direction judging step of judging whether or not the pivotable direction of the second joint is aligned with the movement direction, wherein in the shifting step, the shift to the flexing step may be made when the pivotable direction of the second joint is judged to be aligned with the movement direction in the flexing-direction judging step.

By doing so, it is possible to accurately move the end effector in the input movement direction.

A second aspect of the present invention is a method of controlling a manipulator system that is provided with a manipulator that has an end effector and a joint portion, in this order from a distal-end side, and a manipulation input device with which a movement direction of the end effector can be input, wherein the joint portion has a first joint, which is pivotable about a first axis parallel to a direction in which the end effector and the joint portion are arranged, and a second joint, which is positioned further on the distal-end side than the first joint is and which is pivotable about a second axis perpendicular to the first axis, and wherein the manipulation input device has a manipulation member that can be manipulated by an operator, and the movement direction of the end effector is input by manipulating the manipulation member, the method of controlling the manipulator system including a receiving step of receiving the movement direction from the manipulation input device; a rotating step of actuating the first joint based on the movement direction received in the receiving step; a flexing step of, following the rotating step, actuating the second joint based on the movement direction received in the receiving step; a flexing-direction judging step of judging whether or not the pivotable direction of the second joint is aligned with the movement direction during the rotating step; and a shifting step of shifting from the rotating step to the flexing step when the pivotable direction of the second joint is judged to be aligned with the movement direction in the flexing-direction judging step.

With the second aspect of the present invention, when the actuation of the first joint is started in the rotating step, whether or not the pivoting direction of the second joint is aligned with the movement direction is judged in the flexing-direction judging step, and the procedure shifts to the flexing step in the shifting step when the pivoting direction of the second joint is aligned with the movement direction. By doing so, regardless of the direction of the input movement direction, the end effector can be moved in the movement direction.

In this case, the operator can intuitively move the end effector in a desired direction by a simple manipulation of merely continuing to apply a manipulation corresponding to the movement direction of the end effector to the manipulation member. In addition, because it suffices that the manipulation input device allow at least the movement direction of the end effector to be input, the configuration thereof can be simplified, and the size thereof can be reduced.

The above-described first and second aspects may include a restoring step of restoring the second joint to a predetermined initial position.

By doing so, the second joint can be restored to the predetermined initial position, for example, a position at which the end effector, the first joint, and the second joint are arranged on a straight line.

In the above-described first and second aspects, the restoring step may include a restoring-instruction receiving step of receiving a restoring instruction for instructing the restoration of the second joint to the initial position.

By doing so, the operator can restore the second joint to the predetermined initial position at an arbitrary timing by inputting the restoring instruction.

In the above-described first and second aspects, an actuation speed of the first joint in the rotating step may be greater than an actuating speed of the second joint in the flexing step.

By doing so, the direction of the second joint is quickly aligned and, subsequently, the flexing operation of the second joint is slowly performed. By doing so, it is possible to enhance the usability when performing a treatment, such as making an incision or the like by utilizing the movement of the end effector, by means of the flexing operation of the second joint.

A third aspect of the present invention is a manipulator system including a manipulator provided with an end effector and a joint portion, in this order from a distal-end side, wherein the joint portion has a first joint, which is pivotable about a first axis parallel to a direction in which the end effector and the joint portion are arranged, and a second joint, which is positioned further on the distal-end side than the first joint is and which is pivotable about a second axis perpendicular to the first axis; a manipulation input device that has a manipulation member that can be manipulated by an operator and with which a movement direction of the end effector can be input by manipulating the manipulation member; and a control device that sequentially actuates the first joint and the second joint based on the movement direction input via the manipulation input device, wherein the control device actuates the first joint and, subsequently, actuates the second joint in accordance with changes in a magnitude of a physical amount of the manipulation applied to the manipulation member.

A fourth aspect of the present invention is a manipulator system including a manipulator provided with an end effector and a joint portion, in this order from a distal-end side, wherein the joint portion has a first joint, which is pivotable about a first axis parallel to a direction in which the end effector and the joint portion are arranged, and a second joint, which is positioned further on the distal-end side than the first joint is and which is pivotable about a second axis perpendicular to the first axis; a manipulation input device that has a manipulation member that can be manipulated by an operator and with which a movement direction of the end effector can be input by manipulating the manipulation member; and a control device that sequentially actuates the first joint and the second joint based on the movement direction input via the manipulation input device, wherein the control device actuates the first joint and, subsequently, actuates the second joint when the pivotable direction of the second joint is aligned with the movement direction.

### {Advantageous Effects of Invention}

The present invention affords an advantage in that it is possible to intuitively manipulate an end effector in any direction just by a simple manipulation and it is also possible to reduce the size of a manipulation input device.

### {Brief Description of Drawings}

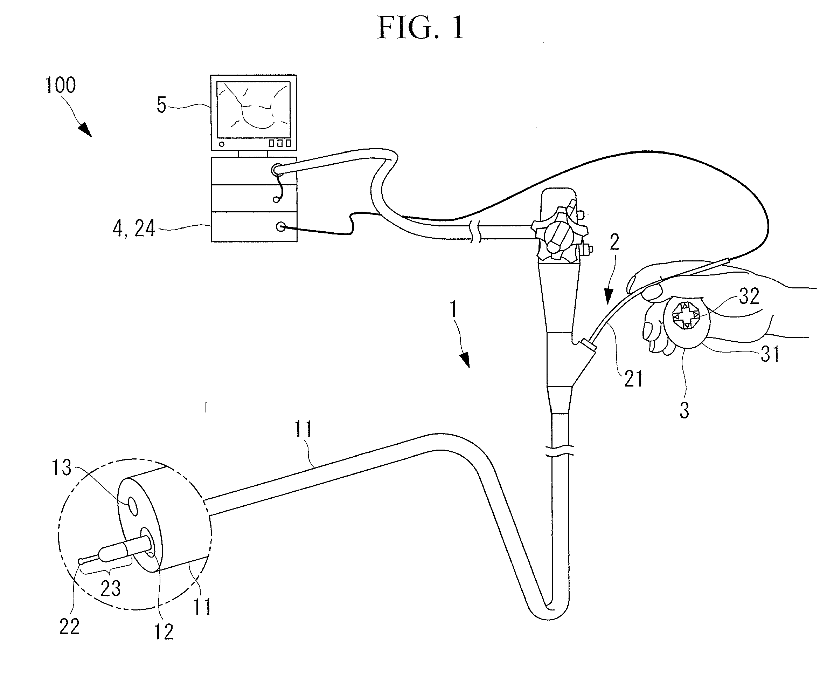

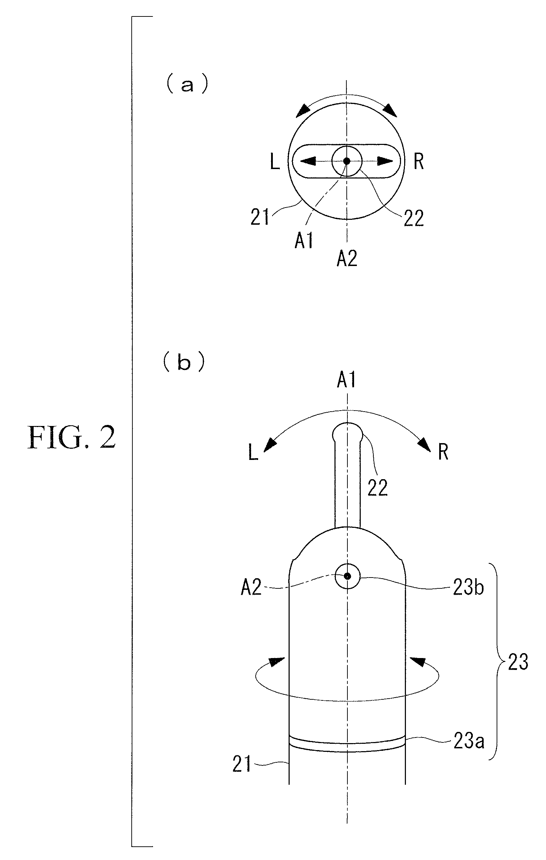

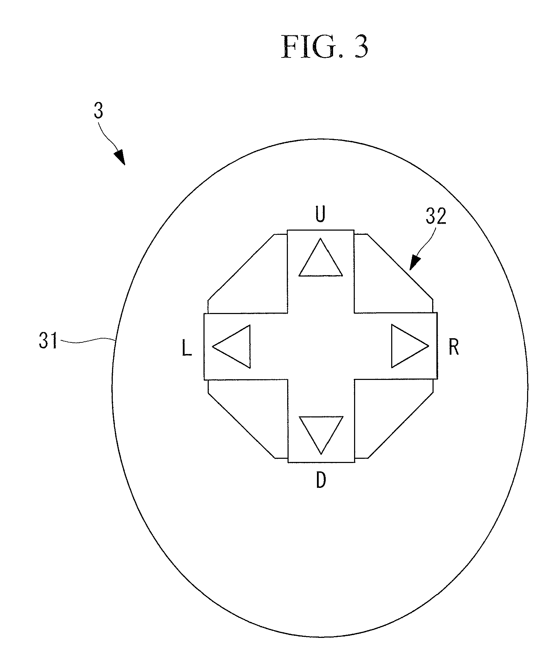

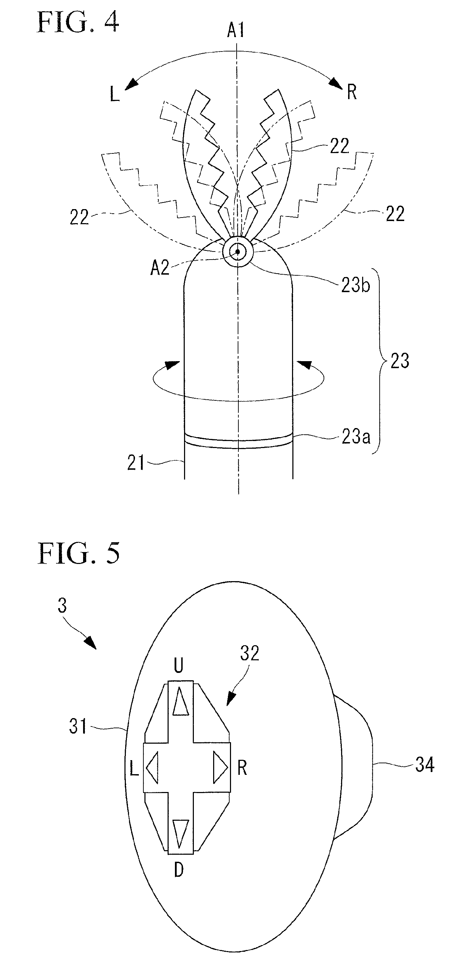

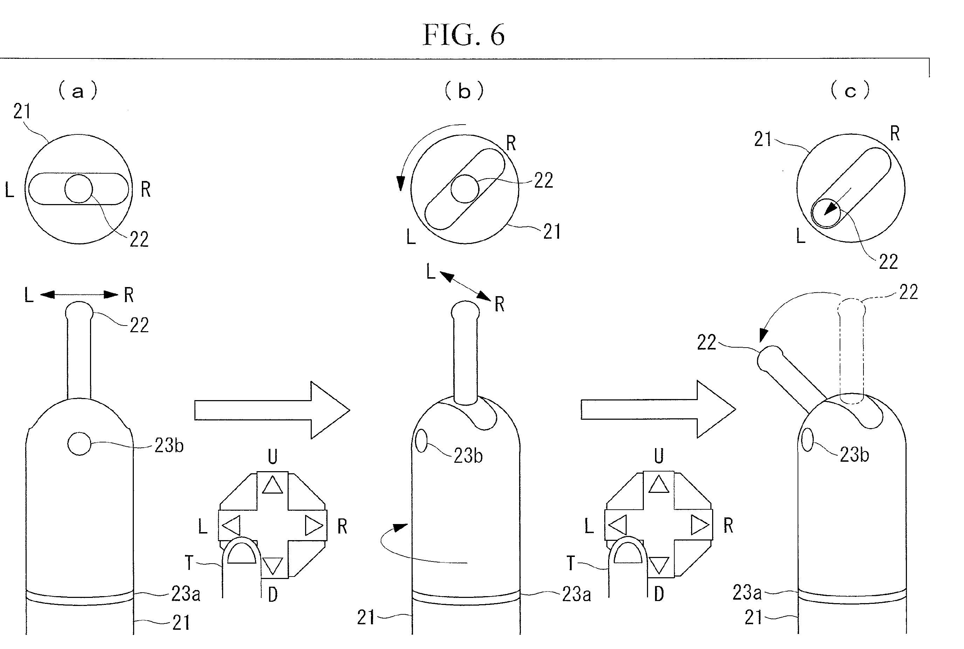

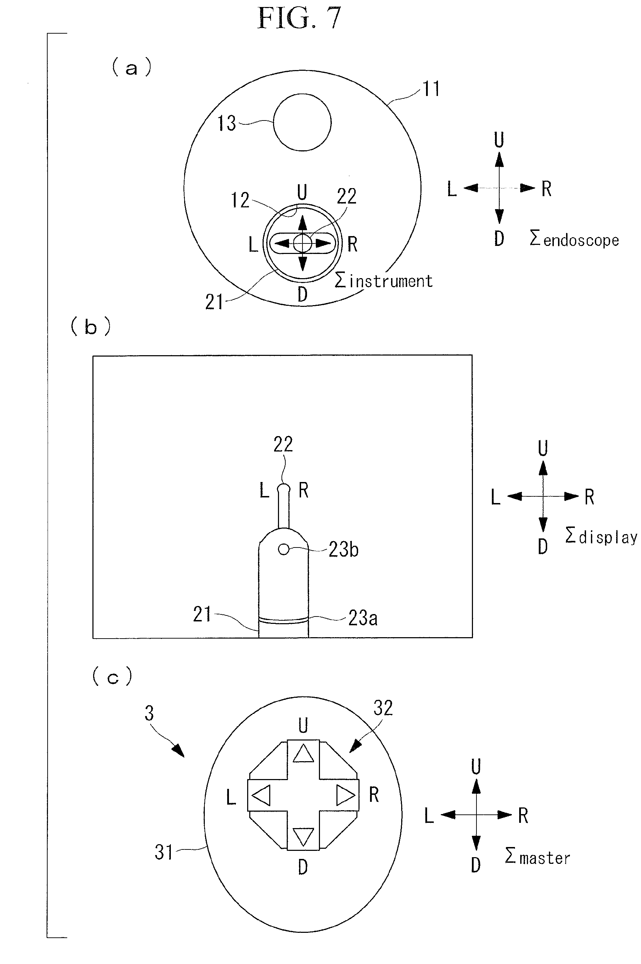

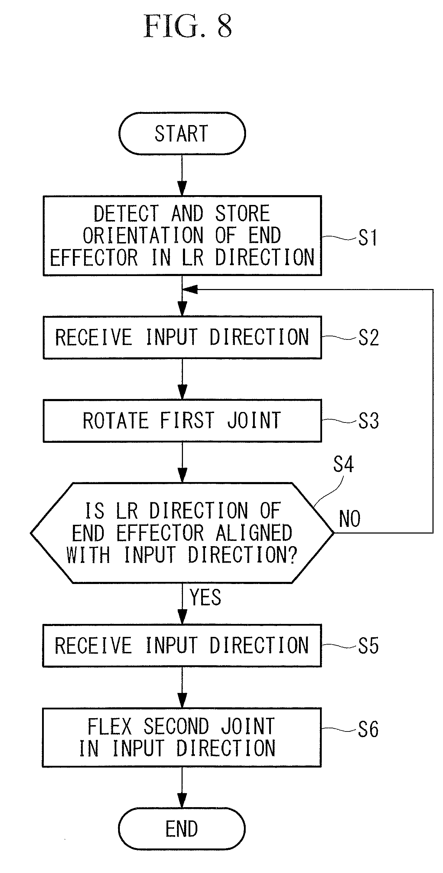

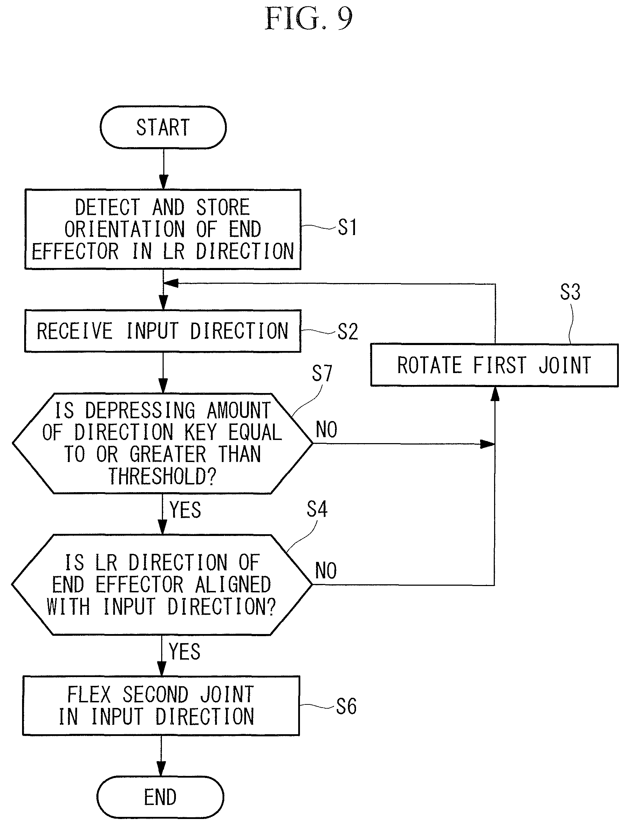

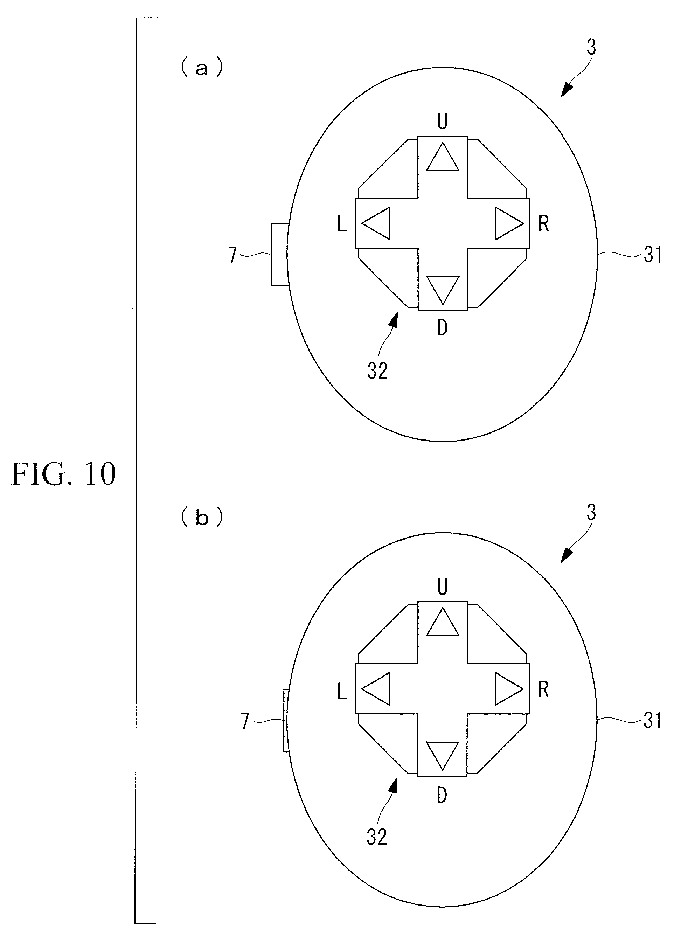

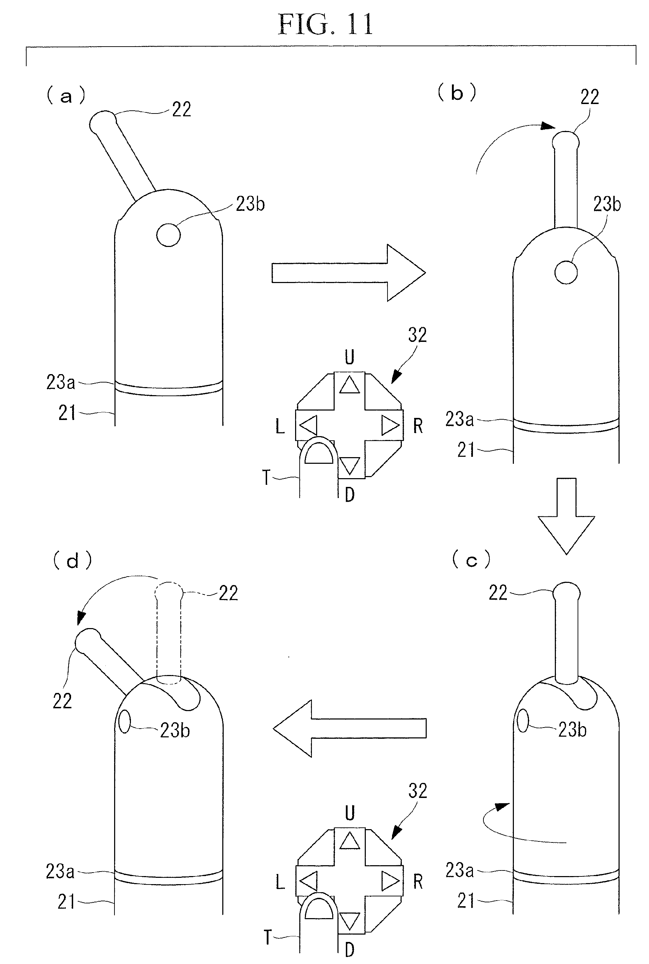

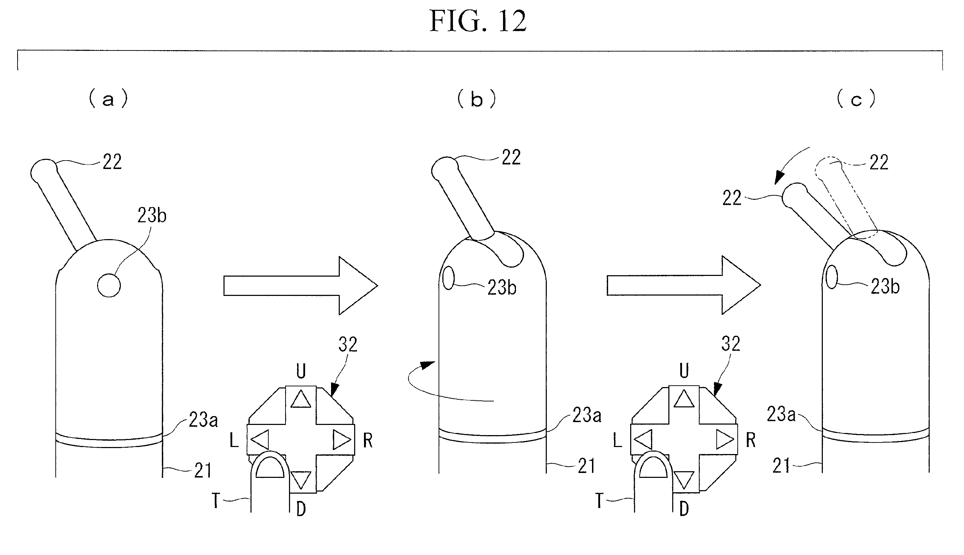

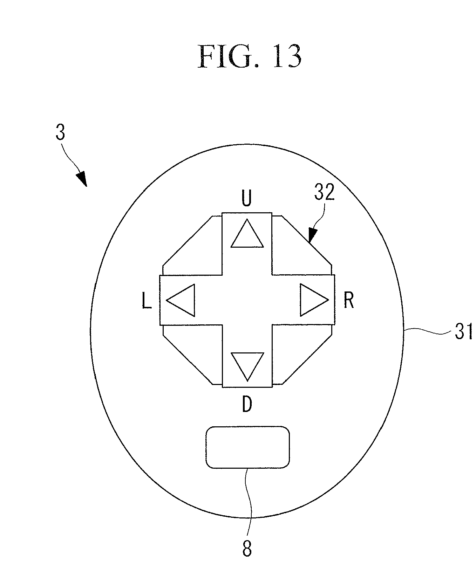

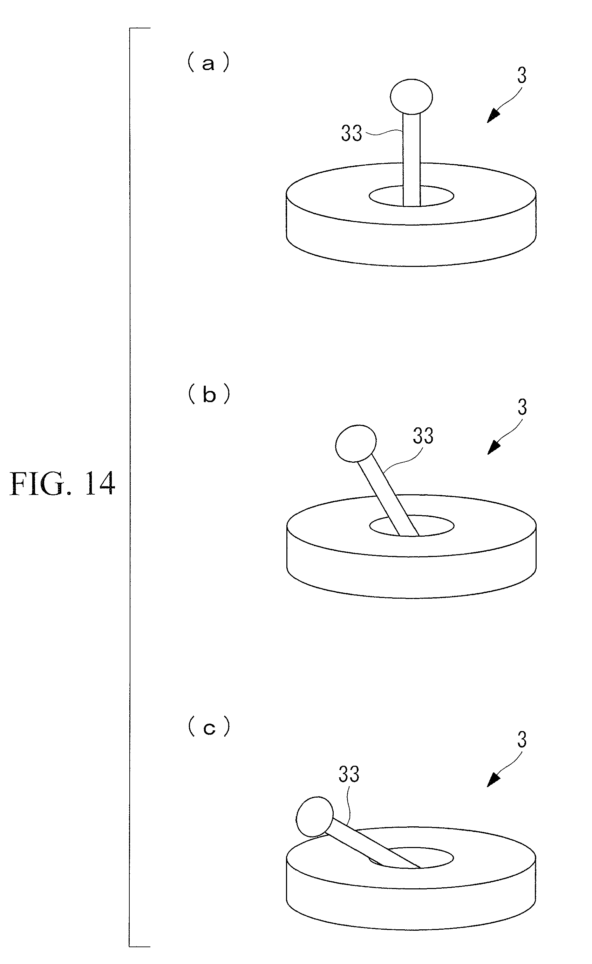

{Fig. 1} Fig. 1 is an overall configuration diagram of a manipulator system according to an embodiment of the present invention.
{Fig. 2} Fig. 2 shows the configurations of an end effector and a joint portion of a treatment tool provided in the manipulator system in Fig. 1, (a) in a front view viewed from the distal-end side and (b) in a side view.
{Fig. 3} Fig. 3 is a front view showing the configuration of a manipulation input device provided in the manipulator system in Fig. 1.
{Fig. 4} Fig. 4 is a side view showing a modification of the end effector and the joint portion in Fig. 2.
{Fig. 5} Fig. 5 is a perspective view showing a modification of the manipulation input device in Fig. 3.
{Fig. 6} Fig. 6 is a diagram for explaining a method of manipulating the end effector in Fig. 3 by using a direction key, showing movements of the joint portion in (a) a receiving step, (b) a rotating step, and (c) a flexing step.
{Fig. 7} Fig. 7 is a diagram for explaining the mutual relationships among (a) the coordinate systems of an inserted portion and a treatment tool of an endoscope, (b) the coordinate system of an endoscope image, and (c) the coordinate system of the direction key.
{Fig. 8} Fig. 8 is a flowchart showing a method of controlling the manipulator system according to the embodiment of the present invention.
{Fig. 9} Fig. 9 is a flowchart showing a modification of the method of controlling the manipulator system in Fig. 8.
{Fig. 10} Figs. 10(a) and (b) are front views of a modification of the manipulation input device provided with a mode-switching button.
{Fig. 11} Fig. 11 is a diagram for explaining a modification of the method of controlling the manipulator system, showing the movements of the joint portion in (a) a receiving step, (b) a restoring step, (c) a rotating step, and (d) a flexing step.
{Fig. 12} Fig. 12 is a diagram for explaining another modification of the method of controlling the manipulator system, showing the movements of the joint portion in (a) a receiving step, (b) a rotating step, and (c) a flexing step.
{Fig. 13} Fig. 13 is a front view of a modification of the manipulation input device provided with a restoring button.
{Fig. 14} Fig. 14 shows a modification of a manipulation input device provided with a joystick, and (a), (b), and (c) are diagrams for explaining the movements of the joystick.

### {Description of Embodiment}

A medical manipulator system 100 according to an embodiment of the present invention will be described below with reference to the drawings.

As shown in Fig. 1, the manipulator system 100 according to this embodiment is provided with a flexible endoscope 1, a treatment tool (manipulator) 2 that can be inserted into a channel 12, which is provided in the endoscope 1, in the longitudinal direction thereof, a manipulation input device 3 with which a surgeon (operator) inputs manipulation instructions for the treatment tool 2, and a control device 4 that controls a joint portion 23 provided in the treatment tool 2.

The endoscope 1 is provided with an elongated inserted portion 11 having flexibility, and the channel 12 that is formed inside the inserted portion 11 so as to pass therethrough in the longitudinal direction. In addition, the endoscope 1 has, at a distal-end surface thereof, a camera 13 that captures, from above, a high-angle image of the treatment tool 2, which protrudes from the exit of the channel 12, and outputs the endoscope image acquired by using the camera 13 to a display portion 5.

Note that, the endoscope 1 is not limited to a flexible one, and a rigid one may be employed depending on the usage thereof.

The treatment tool 2 is provided with an elongated body portion 21 having flexibility, an end effector 22 that is positioned at a distal-end side of the body portion 21 and that treats a portion to be treated, a joint portion 23 that couples the body portion 21 and the end effector 22, and a drive portion 24 that drives the joint portion 23. In Fig. 1, although the drive portion 24 is integrally provided with the control device 4, the drive portion 24 may be separate from the control device 4.

The end effector 22 is a distal-end member that is used in a general surgical treatment tool, for example, forceps, scissors, a needle holder, an electrode, a stapler, or the like.

As shown in Figs. 2(a) and (b), the joint portion 23 has a first joint 23a and a second joint 23b in this order from the base side. The first joint 23a is a rotary joint that is provided so as to be rotatable about a first axis A1, which is coaxial with the center axis of the body portion 21. By rotating the first joint 23a, the end effector 22 can be rotated in a circumferential direction thereof. The second joint 23b is a flexural joint that is provided so as to be pivotable about a second axis A2, which is perpendicular to the first axis A1, and that can be flexed in a direction that intersects the center axis of the body portion 21. By flexing the second joint 23b, the end effector 22 can be moved in a direction that intersects the center axis of the body portion 21. Here, the left-to-right (LR) direction of the end effector 22 is defined, and the second joint 23b and the end effector 22 are provided so as to be movable in the LR direction.

Note that, in this embodiment, although the joint portion 23 provided only with the two joints 23a and 23b will be described in order to simplify the descriptions, the joint portion 23 may be provided with additional joints in addition to these two joints 23a and 23b. For example, a third joint that is pivotable about a third axis, which is perpendicular to the first axis A1, may be provided on the distal-end side of the second joint 23b.

The drive portion 24 receives first control signals and second control signals, described below, from the control device 4, drives the first joint 23a in accordance with the first control signals, and drives the second joint 23b in accordance with the second control signals.

As shown in Fig. 3, the manipulation input device 3 is provided with a main unit 31 that has a shape and a size that allow a surgeon (operator) to grip it with one hand and a direction key (manipulation member) 32 provided in the main unit 31. The direction key 32 is provided at a position that allows manipulations thereof by a thumb T of the one hand holding the main unit 31. The up, down, left, and right directions of the direction key 32 are defined, and the direction key 32 is configured so that inputs can be made in a plurality of two-dimensional directions, or, preferably, in all directions. The manipulation input device 3 transmits the direction in which the direction key 32 is depressed as an input direction (movement direction) to the control device 4 by means of, for example, wireless communication.

Figs. 4 and 5 show modifications of the end effector 22 and the manipulation input device 3. As shown in Fig. 4, the grasping forceps, which is the end effector 22, may be configured so as to be pivoted to both sides thereof. In this case, an open/close switch 34 for opening and closing the grasping forceps is provided in the main unit 31, for example, on the rear side thereof with respect to the direction key 32.

The control device 4 sequentially generates the first control signals and the second control signals based on the input direction received from the manipulation input device 3, and performs two-step control of the joint portion 23 by transmitting the generated control signals to the drive portion 24. Specifically, upon starting to receive the input direction, the control device 4 first generates the first control signals for rotating the first joint 23a so that the LR direction of the end effector 22 in an endoscope image is aligned with the input direction, and causes the first joint 23a to be rotated by transmitting the first control signals to the drive portion 24. After the LR direction of the end effector 22 in the endoscope image is aligned with the input direction, the control device 4 subsequently generates the second control signals for flexing the second joint 23b in the input direction, and causes the second joint 23b to be flexed by transmitting the second control signals to the drive portion 24. Accordingly, by sequentially actuating the first joint 23a and the second joint 23b in association with the passage of time (physical amount) during which the direction key 32 is depressed, the end effector 22 is moved in the direction input via the direction key 32.

For example, as shown in Figs. 6(a) to (c), in the case in which the lower left of the direction key 32 is depressed in a state in which the LR direction of the end effector 22 in the endoscope image is aligned with the LR direction (LR direction of said figures) of the endoscope image (see Fig. 6 (a)), by causing the first joint 23a to be rotated counterclockwise by 45°, the control device 4 directs the LR direction of the end effector 22 in the endoscope image in the diagonal direction that connects the upper right and lower left of the endoscope image (see Fig. 6 (b)). Next, the control device 4 causes the second joint 23b to be flexed in the L direction (see Fig. 6 (c)). Accordingly, the end effector 22 is moved to the lower left as input via the direction key 32.

Here, so long as the input direction is continuously received from the manipulation input device 3, the control device 4 sequentially generates the first control signals and the second control signals in accordance with the above-described procedures. If the transmission of the input directions from the manipulation input device 3 is interrupted, the control device 4 halts the drive portion 24 by stopping the control-signal generation. Specifically, the surgeon can sequentially actuate the first joint 23a and the second joint 23b by continuing to depress the direction key 32 in the same direction, and thus, he/she can move the end effector 22 in a desired direction.

In order to align the direction of the end effector 22 with the input direction, as described above, the control device 4 needs to identify the current LR direction of the end effector 22 in the endoscope image. Figs. 7(a) to (c) show the relationships among a coordinate system ∑_{endoscope} of the inserted portion 11, a coordinate system ∑ᵢₙₛₜᵣᵤₘₑₙₜ of the treatment tool 2 in the inserted portion 11, a coordinate system ∑_{display} of the endoscope image, and a coordinate system ∑ₘₐₛₜₑᵣ of the main unit 31.

The orientation of the end effector 22 in the LR direction in the endoscope image is detected, for example, by using an image-recognition technology, such as detection of the shape of the end effector 22 in the endoscope image, detection of a marker provided in the end effector 22, or the like. Alternatively, initial set-up may be performed so that the LR direction of the end effector 22 in the endoscope image is directed in a predetermined initial directions after inserting the treatment tool 2 into the channel 12, the rotational angle of the first joint 23a may subsequently be detected by using an encoder or the like, and the current LR direction of the end effector 22 may be calculated by adding up the detected rotational angles. Alternatively, instead of performing the initial set-up, the channel 12 and the body portion 21 may be configured to have non-circular cross-sectional shapes, or a structure for preventing the body portion 21 from rotating inside the channel 12 may be provided so that the relative position of the body portion 21 in the circumferential direction with respect to the inserted portion 11 is kept constant.

Next, the operation of the thus-configured manipulator system 100 will be described with reference to Fig. 8.

In order to treat an affected portion by using the manipulator system 100 according to this embodiment, first, the inserted portion 11 is inserted into a body, and the distal end of the inserted portion 11 is placed at a position at which a portion to be treated is observed in an endoscope image displayed on the display portion 5. Next, the treatment tool 2 is inserted into the channel 12. The surgeon can observe the end effector 22 and the joint portion 23 protruding from the exit of the channel 12 in the endoscope image. Regarding the orientation of the end effector 22 in the LR direction in this endoscope image, as described above, a structure that keeps the relative positions of the endoscope 1 and the treatment tool 2 constant is provided, or the orientation of the end effector 22 in the LR direction is detected by means of image recognition or the like, and the detected orientation is stored in the control device 4 (step S1).

Next, the surgeon grips the main unit 31 so that the UDLR direction of the endoscope image and the UDLR direction of the direction key 32 are substantially aligned, and starts to move the end effector 22 by depressing the direction key 32. While the direction key 32 is continuously depressed, the input direction is continuously transmitted to the control device 4 from the manipulation input device 3 (receiving step S2). In accordance with the received input direction, first, the control device 4 causes the first joint 23a to be rotated by generating the first control signals (rotating step S3), thus aligning the LR direction of the end effector 22 with the input direction (flexing-direction judging step S4). After completing the alignment of the direction of the end effector 22 ("YES" in step S4), if the input direction is still continuing to be received (shifting step S5), next, the control device 4 causes the second joint 23b to be flexed in the input direction by generating the second control signals (flexing step S6). By doing so, the end effector 22 is moved in the input direction.

As has been described above, with this embodiment, in the case in which it is not possible to directly move the end effector 22 in the input direction just by flexing the second joint 23b, first, by rotating the first joint 23a, the direction of the second joint 23b is aligned so that the direction in which the second joint 23b can be flexed is aligned with the input direction, and, subsequently, the second joint 23b is flexed in the input direction. At this time, the surgeon does not need to calculate actuation directions and actuation amounts of the individual joints 23a and 23b nor does he/she need to separately manipulate the two joints 23a and 23b, and it suffices that the direction key 32 be continued to be depressed in the direction in which he/she wants to move the end effector 22.

Here, it is preferable that the operation speed of the first joint 23a be greater than the operation speed of the second joint 23b. By doing so, it is possible to quickly align the direction with the input direction, and it is possible to slowly perform the swinging operation of the second joint 23b. Because such a configuration improves the usability, it is suitable when making an incision in tissue by means of the operation of the second joint 23b.

In this way, there is an advantage in that it is possible to intuitively move the end effector 22 in a desired direction just by a single, simple manipulation. In addition, because the surgeon does not need to visually check the direction key 32 at hand frequently, it becomes possible to continue to closely observe the end effector 22 in the endoscope image, and thus, there is an advantage in that it is possible to concentrate his/her attention to manipulation of the end effector 22. Furthermore, the movement direction of the end effector 22 is the only input required to control the joints 23a and 23b, and, because it suffices to provide the direction key 32 having a simple configuration, it is possible to realize a small manipulation input device 3 that can be gripped with one hand.

Note that, although the first joint 23a can be rotated clockwise and counterclockwise, the direction in which the first joint 23a is rotated in the rotating step S3 may be a predefined direction and may be selected depending on situations. For example, of clockwise and counterclockwise directions, the control device 4 may select the direction that requires a smaller amount of rotation. Alternatively, assuming the angle of the first joint 23a to be 0° when the LR direction of the endoscope image is aligned with the LR direction of the end effector 22 in the endoscope image, if the rotational angle of the first joint 23a exceeds 90°, the LR direction of the endoscope image and the LR direction of the end effector 22 in the endoscope image become opposite from each other, and thus, it becomes difficult to intuitively manipulate the end effector 22 while observing the endoscope image. In order to avoid such a situation in advance, the rotation direction of the first joint 23a may be controlled so that the rotational angle of the first joint 23a falls within a range from -90° to +90. In addition, when the rotational angle approaches the operational limit, the first joint 23a may be rotated in the direction that requires a smaller amount of rotation after rotating the first joint 23a by 180°.

In addition, in this embodiment, although the first joint 23a and the second joint 23b are sequentially actuated by holding down the direction key 32, alternatively, the first joint 23a and the second joint 23b may be sequentially actuated by changing the depressing amount (physical amount, amount of movement) of the direction key 32.

For example, with reference to Fig. 9, describing the case of a configuration in which the depressing amount can be changed in two levels by pressing the direction key 32 half way and fully pressing the direction key 32, the surgeon first presses the direction key 32 half way (receiving step S2). In the state in which the direction key 32 is pressed half way ("NO" in shifting step S7), the control device 4 generates the first control signals to rotate the first joint 23a (rotating step S3), thus aligning the direction of the second joint 23b (flexing-direction judging step S4). After the rotation of the first joint 23a is stopped ("YES" in step S4), the surgeon fully presses the direction key 32 (receiving step S2). In the state in which the direction key 32 is fully pressed ("YES" in shifting step S7), the control device 4 generates the second control signals to flex the second joint 23b (flexing step S6), thus moving the end effector 22 in the input direction.

As has been described above, by changing the depressing amount of the direction key 32 also, it is possible to sequentially actuate the first joint 23a and the second joint 23b in a continuous manner while keeping the thumb T placed at the same position of the direction key 32. In addition, unlike the configuration employing the hold-down method described above, the surgeon can decide the timing at which to start flexing the second joint 23b, and thus, the usability can be further enhanced.

If the direction key 32 is unintentionally fully pressed before direction alignment of the second joint 23b is completed, the situation can be resolved by using any one of first to third methods described below. One of the first to third methods described below may be made selectable for the surgeon.

In the first method, the first joint 23a is continued to be rotated until the direction alignment of the second joint 23b is completed, and the second joint 23b is flexed after completing the direction alignment.

In the second method, rotation of the first joint 23a is stopped at the point in time at which the direction key 32 is fully pressed, and flexing of the second joint 23b is started.

In the third method, the actuation is aborted for both joints 23a and 23b, and a notification signal that indicates incomplete direction alignment of the second joint 23b is output. Aborting can be canceled, for example, by pressing the direction key 32 half way in an arbitrary direction.

In addition, as shown in Figs. 10(a) and (b), in this embodiment, the main unit 31 may be provided with a mode-switching switch 7 with which a rotating mode for rotating the first joint 23a or a flexing mode for flexing the second joint 23b is alternatively selected. This mode-switching switch 7 is provided at a position that allows manipulation thereof by a finger other than the thumb T, and the surgeon can switch between the modes while keeping the thumb T placed on the direction key 32.

The mode-switching switch 7 shown in Figs. 10(a) and (b) employs a push-button switch, and when the mode-switching switch 7 is not depressed, as shown in Fig. 10(a), the control device 4 selects the rotating mode and rotates the first joint 23a when the direction key 32 is depressed. On the other hand, when the mode-switching switch 7 is depressed, as shown in Fig. 10(b), the control device 4 selects the flexing mode and flexes the second joint 23b when the direction key 32 is depressed. However, in the flexing mode, the control device 4 judges whether or not the LR direction of the end effector 22 in the endoscope image is aligned with the input direction, and actuates the second joint 23b only when the directions are aligned.

As has been described above, by providing the mode-switching switch 7 also, it is possible to intuitively manipulate the end effector 22 while keeping the thumb T placed on the same position of the direction key 32.

In addition, in this embodiment, when another direction is input via the direction key 32 in a state in which the second joint 23b is flexed, as shown in Figs. 11(a) to (d), the rotating step S3 and the steps thereafter (see Figs. 11(c) and (d)) may be executed after executing a restoring step (see Fig. 11(b)) of restoring the second joint 23b to an initial position at which the end effector 22 and the body portion 21 are arranged on a straight line (i.e., at 0°).

When rotating the first joint 23a in a state in which the second joint 23b is flexed, the movement range of the end effector 22 is increased. Therefore, by restoring the second joint 23b to the initial position by executing the restoring step before rotating the first joint 23a, it is possible to smoothly move the end effector 22 in a small space.

In addition, as shown in Figs. 12(a) to (c), the rotating step S3 and the steps thereafter may be executed while the second joint 23b is kept in the flexed state.

Furthermore, it is permissible to employ a configuration in which a first mode, in which the rotating step S3 and the steps thereafter are executed after executing the restoring step, and a second mode, in which the restoring step is omitted and the rotating step S3 and the steps thereafter are executed while the second joint 23b is kept flexed, are switched depending on the situation. In the case in which an external force equal to or greater than a predetermined value is exerted on the end effector 22, for example, when the end effector 22 is in contact with tissue, the first mode may be selected, and when the magnitude of the external force is less than the predetermined value, the second mode may be selected.

This mode switching may be automatically performed by the control device 4, and, for example, when an external force equal to or greater than the predetermined value is exerted on the end effector 22, an alert for prompting the surgeon to switch to the first mode may be output. In addition, in the second mode, only the rotating step S3 may be executed, while prohibiting the flexing step S6. Alternatively, a means (for example, a switch similar to the mode-switching switch 7 in Fig. 10) for alternatively selecting the first mode or the second mode may be provided in the main unit 31, and the configuration thereof may be such that the surgeon can select one of the first mode and the second mode.

In addition, in this embodiment, a restoring button 8 may be provided in the main unit 31, as shown in Fig. 13. It is preferable that this restoring button 8 also be provided at a position in the main unit 31 that allows the operator to perform manipulation thereof by a finger other than the thumb T. In this case, the control device 4 receives a restoring instruction from the manipulation input device 3 when the restoring button 8 is depressed (restoring-instruction receiving step), and forcedly restores at least the second joint 23b to the initial position based on the restoring instruction.

By doing so, the surgeon can restore the second joint 23b to the initial position at an arbitrary timing. In this case, not only the second joint 23b, but also the first joint 23a may be configured so as to be restored to a predetermined initial position (for example, a position at which the LR direction of the endoscope image is aligned with the LR direction of the end effector 22 in the endoscope image).

In addition, although the direction key 32 has been described as the manipulation member in this embodiment, alternatively, as shown in Figs. 14(a) to (c), a joystick 33 that can be tilted in a plurality of two-dimensional directions, or, preferably, in all directions, may be employed.

In this case, in correspondence with the half pressing and full pressing of the direction key 32, the control device 4 shifts the procedure from the rotating step S3 to the flexing step S6 in accordance with changes in the tilting angle (physical amount, amount of movement) of the joystick 33. For example, the joystick 33 is configured so that the tilting angle thereof can be changed between a shallow first angle (see Fig. 14(b)) and a deep second angle (see Fig. 14(c)). The control device 4 may be configured so as to execute the rotating step S3 when the tilting angle of the joystick 33 is at the first angle and to execute the flexing step S6 when the tilting angle of the joystick 33 is at the second angle.

By doing so also, the surgeon can intuitively move the end effector 22 in an arbitrary desired direction by means of simple manipulations of merely changing the tilting angle after tilting the joystick 33 in one direction.

### {Reference Signs List}

- 1: endoscope
- 11: inserted portion
- 12: channel
- 13: camera
- 2: treatment tool (manipulator)
- 21: body portion
- 22: end effector
- 23: joint portion
- 23a: first joint
- 23b: second joint
- 24: drive portion
- 3: manipulation input device
- 31: main unit
- 32: direction key (manipulation member)
- 33: joystick (manipulation member)
- 4: control device
- 5: display portion
- 7: mode-switching switch
- 8: restoring button
- 100: manipulator system
- A1: first axis
- A2: second axis
- T: thumb
- S2, S5: receiving step
- S3: rotating step
- S4: flexing-direction judging step
- S5, S7: shifting step
- S6: flexing step

## Claims

1. A method of controlling a manipulator system that is provided with a manipulator that has an end effector and a joint portion, in this order from a distal-end side, and a manipulation input device with which a movement direction of the end effector can be input, wherein the joint portion has a first joint, which is pivotable about a first axis parallel to a direction in which the end effector and the joint portion are arranged, and a second joint, which is positioned further on the distal-end side than the first joint is and which is pivotable about a second axis perpendicular to the first axis, and wherein the manipulation input device has a manipulation member that can be manipulated by an operator, and the movement direction of the end effector is input by manipulating the manipulation member, the method of controlling the manipulator system comprising:
a receiving step of receiving the movement direction from the manipulation input device;
a rotating step of actuating the first joint based on the movement direction received in the receiving step;
a flexing step of, following the rotating step, actuating the second joint based on the movement direction received in the receiving step; and
a shifting step of shifting from the rotating step to the flexing step in accordance with changes in a magnitude of a physical amount of a manipulation applied to the manipulation member.

2. The method of controlling a manipulator system according to Claim 1, wherein
in the receiving step, the movement direction corresponding to a two-dimensional position at which the manipulation is applied to the manipulation member or a direction in which the manipulation is applied to the manipulation member is received, and
in the shifting step, the shift to the flexing step from the rotating step is made in accordance with changes in the physical amount of the manipulation applied at the same position or in the same direction.

3. The method of controlling a manipulator system according to Claim 2, wherein the physical amount is an amount of time during which the manipulation member is being manipulated or an amount of movement of the manipulation member.

4. The method of controlling a manipulator system according to any one of Claims 1 to 3, further comprising:
a flexing-direction judging step of judging whether or not the pivotable direction of the second joint is aligned with the movement direction,
wherein in the shifting step, the shift to the flexing step is made when the pivotable direction of the second joint is judged to be aligned with the movement direction in the flexing-direction judging step.

5. A method of controlling a manipulator system that is provided with a manipulator that has an end effector and a joint portion, in this order from a distal-end side, and a manipulation input device with which a movement direction of the end effector can be input, wherein the joint portion has a first joint, which is pivotable about a first axis parallel to a direction in which the end effector and the joint portion are arranged, and a second joint, which is positioned further on the distal-end side than the first joint is and which is pivotable about a second axis perpendicular to the first axis, and wherein the manipulation input device has a manipulation member that can be manipulated by an operator, and the movement direction of the end effector is input by manipulating the manipulation member, the method of controlling the manipulator system comprising:
a receiving step of receiving the movement direction from the manipulation input device;
a rotating step of actuating the first joint based on the movement direction received in the receiving step;
a flexing step of, following the rotating step, actuating the second joint based on the movement direction received in the receiving step;
a flexing-direction judging step of judging whether or not the pivotable direction of the second joint is aligned with the movement direction during the rotating step; and
a shifting step of shifting from the rotating step to the flexing step when the pivotable direction of the second joint is judged to be aligned with the movement direction in the flexing-direction judging step.

6. The method of controlling a manipulator system according to any one of Claims 1 to 5, further comprising:
a restoring step of restoring the second joint to a predetermined initial position.

7. The method of controlling a manipulator system according to Claim 6, wherein the restoring step includes a restoring-instruction receiving step of receiving a restoring instruction for instructing the restoration of the second joint to the initial position.

8. The method of controlling a manipulator system according to any one of Claims 1 to 7, wherein an actuation speed of the first joint in the rotating step is greater than an actuating speed of the second joint in the flexing step.

9. A manipulator system comprising:
a manipulator provided with an end effector and a joint portion, in this order from a distal-end side, wherein the joint portion has a first joint, which is pivotable about a first axis parallel to a direction in which the end effector and the joint portion are arranged, and a second joint, which is positioned further on the distal-end side than the first joint is and which is pivotable about a second axis perpendicular to the first axis;
a manipulation input device that has a manipulation member that can be manipulated by an operator and with which a movement direction of the end effector can be input by manipulating the manipulation member; and
a control device that sequentially actuates the first joint and the second joint based on the movement direction input via the manipulation input device,
wherein the control device actuates the first joint and, subsequently, actuates the second joint in accordance with changes in a magnitude of a physical amount of the manipulation applied to the manipulation member.

10. A manipulator system comprising:
a manipulator provided with an end effector and a joint portion, in this order from a distal-end side, wherein the joint portion has a first joint, which is pivotable about a first axis parallel to a direction in which the end effector and the joint portion are arranged, and a second joint, which is positioned further on the distal-end side than the first joint is and which is pivotable about a second axis perpendicular to the first axis;
a manipulation input device that has a manipulation member that can be manipulated by an operator and with which a movement direction of the end effector can be input by manipulating the manipulation member; and
a control device that sequentially actuates the first joint and the second joint based on the movement direction input via the manipulation input device,
wherein the control device actuates the first joint and, subsequently, actuates the second joint when the pivotable direction of the second joint is aligned with the movement direction.
